# DEMANDE DE BREVET EUROPEEN

(11) **EP 4 813 354 A1**
(43) Date de publication de la demande: **30.09.2026**
(21) Numéro de dépôt: 26152909.3
(22) Date de dépôt: 20.01.2026
(51) Int. Cl.: A61F 5/02

(54) **CEINTURE DE SOUTIEN LOMBAIRE**

(30) Priorité: 24.03.2025 FR 2502990
(71) Demandeur: LABORATOIRES INNOTHERA, 94110 Arcueil (FR)
(72) Inventeur: DUPORT, Guillaume, 42000 SAINT ETIENNE (FR)
(74) Mandataire: Ipsilon

(57) **Abrégé**

La ceinture (1) comprend :
- deux branches (6, 7) possédant chacune une partie principale (12) élastiquement extensible et un dossard (10), les dossards (10) étant des pièces distinctes ;
- un câble de serrage (30) non-extensible qui présente une portion centrale de laçage (31) reliant les dossards (10) ainsi que deux portions latérales (32), une traction longitudinale exercée sur chaque portion latérale (32), dans des sens opposés, conduisant au rapprochement mutuel des dossards (10) ;
- un dispositif de liaison (50) des dossards (10) entre eux, distinct du câble de serrage (30), agencé et fixé entre les dossards (10).

Le dispositif de liaison (50) est élastiquement déformable selon l'axe longitudinal (3) et possède selon l'axe médian (2) une nervosité suffisamment importante pour empêcher le décalage d'un dossard (10) par rapport à l'autre.

## Description

L'invention concerne une ceinture de soutien lombaire.

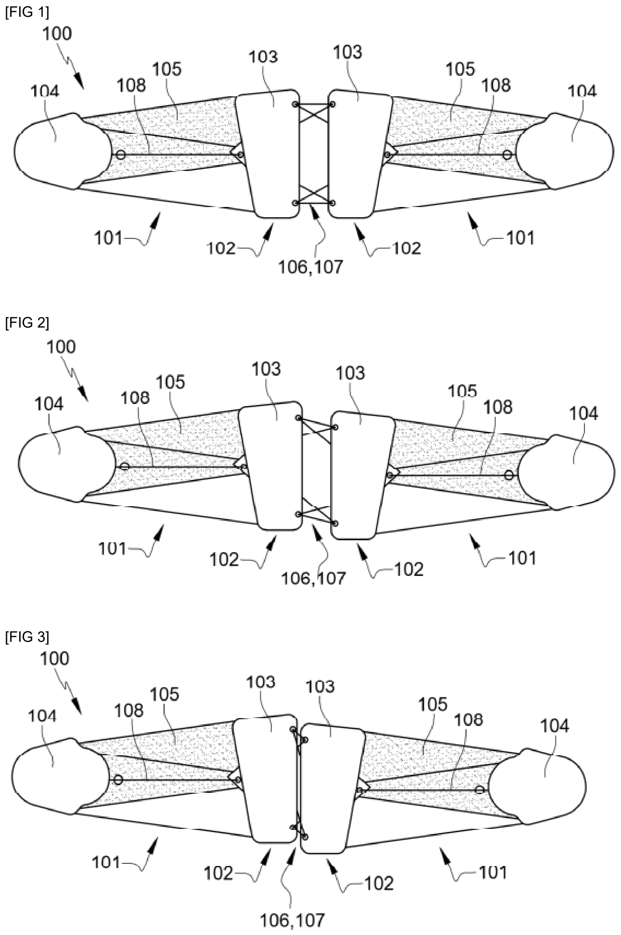

L'invention se rapporte plus particulièrement à une ceinture de soutien lombaire telle qu'illustrée schématiquement sur la figure 1. Une telle ceinture 100 comporte deux branches 101 présentant chacune une partie extrême médiale 102 comportant un dossard 103 destiné à être placé contre le dos du porteur, une partie extrême latérale 104, et une partie principale 105 qui est élastiquement extensible longitudinalement et qui relie le dossard 103 et la partie extrême latérale 104. Les dossards 103 des deux branches 101 sont des pièces distinctes.

Une ceinture 100 de ce type comprend également un câble de serrage 106 qui est non-extensible. Le câble de serrage 106 présente une portion centrale de laçage 107 qui relie les deux dossards 103 et qui est prolongée de part et d'autre par une portion latérale 108.

Sur la figure 1, la ceinture 100 est représentée à plat, à l'état non contraint.

Pour la mise en place de la ceinture 100, sur laquelle les dossards 103 sont espacés l'un de l'autre, un porteur positionne les branches 101 de part et d'autre de la partie inférieure de son tronc, place les parties extrêmes latérales 104 des branches 101 l'une sur l'autre en regard de sa zone abdominale et assemble ces parties extrêmes latérales 104 l'une à l'autre grâce à des moyens d'accrochage complémentaires.

Le porteur va ensuite procéder au serrage approprié de la ceinture 100. Pour cela, il va exercer une traction sensiblement longitudinale sur chaque portion latérale 108 du câble de serrage 106, dans des sens opposés. Il s'ensuit que, grâce à la portion centrale de laçage 107, les dossards 103 se rapprochent l'un de l'autre, c'est-à-dire que l'écartement longitudinal entre eux diminue.

Cependant, ce serrage se traduit très souvent par le décalage vertical entre les deux dossards 103. Ce problème peut être dû à différents facteurs, tels qu'un effort de traction exercé sur le câble de serrage avec une amplitude différente à gauche et à droite, à une posture non symétrique du porteur lors du serrage, à la morphologie du porteur, etc.

Le décalage vertical entre les deux dossards 103 au cours du serrage est illustré schématiquement sur les figures 2 et 3. En fin d'opération de serrage, la ceinture 100 peut ainsi se trouver dans la configuration de la figure 3. Un tel décalage des dossards 103 l'un par rapport à l'autre, dans la direction verticale en position d'utilisation de la ceinture, est préjudiciable à la qualité du soutien apporté par la ceinture et est de plus source d'inconfort.

L'invention vise à remédier à ce problème.

A cet effet, l'invention concerne une ceinture de soutien lombaire présentant un axe médian de symétrie et un axe longitudinal orthogonal à l'axe médian, la ceinture comportant deux branches destinées à être positionnées de part et d'autre de la partie inférieure du tronc d'un porteur, chaque branche comprenant une partie extrême médiale comportant un dossard destiné à être placé contre le dos du porteur, une partie extrême latérale, et une partie principale qui est élastiquement extensible longitudinalement et qui relie le dossard et la partie extrême latérale, les dossards des deux branches étant des pièces distinctes, les parties extrêmes latérales étant pourvues de moyens d'accrochage complémentaires pour pouvoir être assemblées lorsqu'elles sont placées l'une sur l'autre en regard de la zone abdominale du porteur. La ceinture comprend en outre un câble de serrage, ledit câble de serrage étant non-extensible et présentant d'une part une portion centrale de laçage qui relie les deux dossards et d'autre part deux portions latérales reliées chacune à l'une des extrémités latérales de la portion centrale de laçage, une traction sensiblement longitudinale exercée sur chaque portion latérale, dans des sens opposés, conduisant au rapprochement mutuel des dossards.

Selon une définition générale de l'invention, la ceinture comprend en outre un dispositif de liaison des dossards entre eux, distinct du câble de serrage, ledit dispositif de liaison étant agencé entre les dossards et fixé à chacun des dossards, ledit dispositif de liaison étant élastiquement déformable selon l'axe longitudinal et possédant dans la direction orthogonale à l'axe longitudinal une nervosité suffisamment importante pour empêcher sensiblement le décalage d'un dossard par rapport à l'autre parallèlement à l'axe médian de la ceinture.

Ainsi, de par son élasticité, le dispositif de liaison n'empêche ni l'écartement mutuel des dossards ni un éventuel allongement longitudinal de la ceinture lorsque celle-ci est mise en place autour du tronc d'un porteur. De préférence, la présence additionnelle du dispositif de liaison ne se traduit pas par une augmentation sensible de la force requise pour la mise en place et le réglage en traction de la ceinture.

Par ailleurs, lors d'une traction sur le câble de serrage conduisant au rapprochement des dossards, les sollicitations s'exerçant sur le dispositif de liaison diminuent, et, par élasticité, le dispositif de liaison tend à reprendre la forme et les dimensions qu'il avait avant d'être soumis à ces sollicitations. Ce retour vers l'état non contraint du dispositif de liaison s'effectue sans déformation sensible du dispositif de liaison selon la direction orthogonale à l'axe longitudinal, du fait de la nervosité du dispositif de liaison dans cette direction. La nervosité correspond à une résistance à la déformation élastique, à une valeur quantifiant la capacité du dispositif de liaison à résister à la déformation, c'est-à-dire à la force nécessaire pour obtenir la déformation. En conséquence, la traction sur le câble de serrage n'entraîne pas un déplacement des dossards l'un par rapport à l'autre selon la direction orthogonale à l'axe longitudinal.

La fonction du dispositif de liaison est de contrer la force de pesanteur s'exerçant sur un dossard, et non de rapprocher les dossards l'un de l'autre pour obtenir le serrage approprié de la ceinture.

Le maintien de la position relative des dossards, sensiblement sans décalage de l'un par rapport à l'autre le long de l'axe médian de la ceinture, assure un positionnement approprié de ces dossards et un confort de port de la ceinture.

De préférence, les deux dossards sont liés l'un à l'autre uniquement par le câble de serrage et le dispositif de liaison, et par aucun autre élément.

La nervosité du dispositif de liaison est de préférence inférieure à la nervosité de chacune des branches. Par exemple, la nervosité de chaque branche est comprise entre 350 et 450 cN/cm à 30% d'allongement, et la nervosité du dispositif de liaison est comprise entre 50 et 150 cN/cm à 30% d'allongement.

Selon une réalisation possible, le dispositif de liaison peut passer :
- d'un état non contraint, dans lequel les dossards sont espacés selon l'axe longitudinal d'une première distance d1 ;
- à un état étendu, à la suite d'un écartement mutuel des dossards selon l'axe longitudinal, état étendu dans lequel les dossards sont espacés selon l'axe longitudinal d'une deuxième distance d2 supérieure à la première distance d1, le dispositif de liaison étant élastiquement déformé,
- puis à un état serré, à la suite d'une traction sensiblement longitudinale exercée sur les portions latérales du câble de serrage, dans des sens opposés, état serré dans lequel les dossards sont espacés selon l'axe longitudinal d'une troisième distance d3 telle que d1 ≤ d3 < d2.

Par « état non contraint », on entend un état où aucune action extérieure n'est appliquée à la ceinture ni au dispositif de liaison, en particulier aucune traction d'un utilisateur, que ce soit sur les branches ou sur le câble de serrage. Dans cet état non contraint, le dispositif de liaison est au repos, non étendu.

A l'état étendu, l'extrémité latérale de chacune des portions latérales du câble de serrage peut être fixée, de façon détachable, à la partie extrême latérale de la branche correspondante.

Après avoir détaché les extrémités latérales des portions latérales du câble de serrage et les avoir tirées, le dispositif de liaison est à l'état serré, où il peut être plus ou moins déformé longitudinalement selon le serrage appliqué. Lesdites extrémités latérales sont alors à nouveau fixées, de façon détachable, à la partie extrême latérale des branches.

Le dispositif de liaison peut être agencé du côté extérieur de la ceinture, c'est-à-dire le côté tourné à l'opposé du porteur, en position d'utilisation.

Par exemple, le dispositif de liaison est agencé sensiblement dans la partie centrale de la ceinture, selon l'axe médian.

La portion centrale de laçage du câble de serrage peut comporter deux parties agencées de part et d'autre du dispositif de liaison, selon l'axe médian.

Le dispositif de liaison peut s'étendre sur une hauteur comprise entre 25 % et 45 % de la hauteur des dossards, le long de l'axe médian, par exemple de l'ordre du tiers de la hauteur des dossards.

Selon un premier mode de réalisation, le dispositif de liaison comprend deux sangles allongées indépendantes l'une de l'autre, chaque sangle étant disposée en oblique et possédant une première extrémité fixée à l'un des dossards et une deuxième extrémité fixée à l'autre des dossards, les deux sangles formant une croix. De préférence, les sangles sont identiques et disposées symétriquement, le point central de la croix étant agencé à l'intersection de l'axe longitudinal et de l'axe médian de la ceinture, et ce quelle que soit l'espacement longitudinal entre les dossards.

Selon un deuxième mode de réalisation, le dispositif de liaison comprend un élément globalement rectangulaire possédant, à l'état non contraint, deux grands côtés qui s'étendent sensiblement orthogonalement à l'axe longitudinal et qui sont fixés chacun à un dossard.

De préférence, la ceinture comprend un unique élément de ce type liant les dossards. Par exemple, à l'état non contraint, la longueur de l'élément est d'au moins 1,5 voire au moins 2 fois sa largeur.

Selon une réalisation possible, la portion centrale de laçage du câble de serrage comprend au moins un brin longitudinal passant dans un œillet ménagé dans chacun des dossards et un brin oblique s'étendant depuis un œillet ménagé dans le dossard d'une branche en direction de la portion latérale du câble de serrage qui est située en regard de la partie principale de l'autre branche.

Par « œillet », on entend un petit trou pratiqué dans le dossard ou tout autre élément de guidage recevant le câble et permettant de changer sa direction.

On peut prévoir que la portion centrale de laçage présente une symétrie par rapport à l'axe longitudinal et par rapport à l'axe médian de la ceinture. De préférence, la portion centrale de laçage forme une boucle fermée. Entre ledit brin longitudinal et ledit brin oblique, le câble de serrage peut former plusieurs zigzags ou aucun.

Selon une réalisation possible, la partie principale de chaque branche comporte deux bandes élastiques qui sont superposées, sur une partie de leur surface, selon la direction antéro-postérieure. Par exemple, pour chaque branche, la portion latérale du câble de serrage peut être agencée entre les deux bandes élastiques selon la direction antéro-postérieure, à l'exception d'une partie extrême latérale du câble de serrage qui traverse une bande pour être située du côté extérieur de la ceinture.

Le câble de serrage se trouve ainsi majoritairement masqué. Au-delà de l'aspect esthétique, une telle configuration évite que le câble de serrage ne s'accroche dans des objets environnants, ce qui est désagréable et risque d'endommager la ceinture.

On décrit à présent, à titre d'exemples non limitatifs, plusieurs modes de réalisation possibles de l'invention, en référence aux figures annexées :
[FIG 1], [FIG 2] et [FIG 3] sont des vues en plan d'une ceinture de l'art antérieur à des étapes successives de traction d'un câble de serrage.
[FIG 4] est une vue en plan d'une ceinture selon un mode de réalisation de l'invention, du côté extérieur, la ceinture comportant un dispositif de liaison qui est à l'état non contraint.
[FIG 5] est une vue en plan de la ceinture de la figure 4, du côté intérieur.
[FIG 6] est une vue similaire à la figure 4, le dispositif de liaison étant à l'état étendu.
[FIG 7] est une vue similaire aux figures 4 et 5, le dispositif de liaison étant à l'état serré.
[FIG 8] est une vue en plan d'une ceinture selon un autre mode de réalisation de l'invention, du côté extérieur, la ceinture comportant un dispositif de liaison qui est à l'état étendu.
[FIG 9] est une vue similaire à la figure 8, le dispositif de liaison étant à l'état serré.

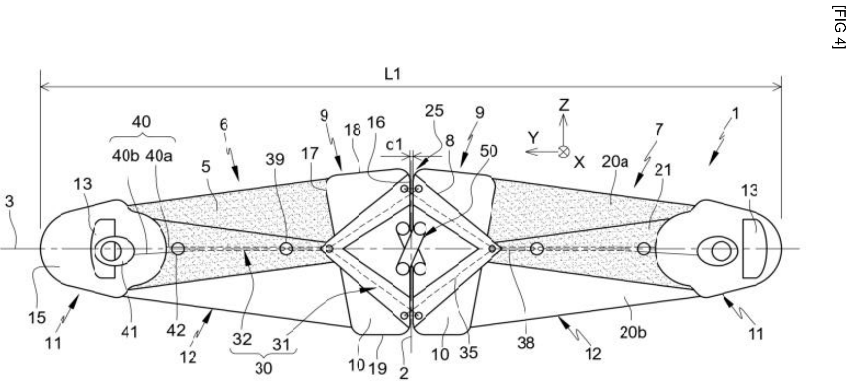

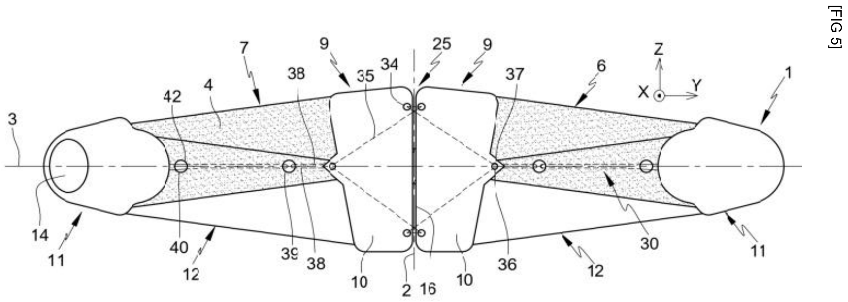

Les figures 4 et 5 illustrent une ceinture de soutien lombaire 1 selon un mode de réalisation de l'invention, la ceinture étant posée à plat et étant à l'état non contraint, c'est-à-dire en particulier qu'aucune traction extérieure n'est exercée sur elle ni sur l'un de ses composants.

La ceinture 1 présente un axe médian 2 de symétrie et un axe longitudinal 3 orthogonal à l'axe médian 2. Dans l'exemple de réalisation représenté, l'axe longitudinal 3 est un axe de symétrie de la ceinture, ceci n'étant pas limitatif.

Comme illustré sur les figures 4 et 5, en référence à un porteur de la ceinture 1, et conformément au système de référence anatomique, on définit la direction Z comme la direction verticale, la direction X comme la direction antéro-postérieure, et la direction Y comme la direction horizontale orthogonale à X.

Ainsi, lorsque la ceinture 1 est portée, l'axe médian 2 est sensiblement vertical, et l'axe longitudinal 3 - alors enroulé autour du porteur- est sensiblement horizontal.

Les termes « haut », « bas », « hauteur », « inférieur », « supérieur » et analogue sont employés en référence à la direction Z.

Les termes « gauche », « droit », « médial » et « latéral » sont employés en référence à la direction Y. Un élément sera qualifié de « médial » s'il est situé plus près de l'axe médian 2 qu'un autre élément, alors qualifié de « latéral ».

Les termes « antérieur », « postérieur » et analogue, sont employés en référence à la direction X.

La ceinture 1 présente une face intérieure 4, visible sur la figure 5, qui est tournée vers le porteur en position d'utilisation, et une face extérieure 5 opposée, qui est tournée à l'opposé du porteur en position d'utilisation, et qui est visible sur la figure 4.

La ceinture 1 comprend deux branches destinées à être positionnées de part et d'autre de la partie inférieure du tronc d'un porteur, à savoir une branche gauche 6 et une branche droite 7. Chaque branche 6, 7 comprend une partie extrême médiale 9 qui comporte un dossard 10 destiné à être placé contre le dos du porteur, une partie extrême latérale 11, et une partie principale 12 qui est élastiquement extensible longitudinalement et qui relie le dossard 10 et la partie extrême latérale 11.

Les parties extrêmes latérales 11 sont pourvues de moyens d'accrochage complémentaires pour pouvoir être assemblées lorsqu'elles sont placées l'une sur l'autre en regard de la zone abdominale du porteur. L'accrochage peut s'effectuer au moyen d'un système de type Velcro^{®}. A cet effet, par exemple, la partie extrême latérale 11 d'une branche 7 peut être pourvue sur sa face intérieure 4 d'une zone 14 de crochets et la partie extrême latérale 11 de l'autre branche 6 peut être pourvue sur sa face extérieure 5 d'une zone 15 de boucles ou d'un tissu pouvant coopérer avec les crochets, comme de l'astrakan. Pour faciliter la mise en place de la ceinture 1 et la traction sur les branches 6, 7, l'une ou chacune des parties extrêmes latérales 11 peut comporter un passe-doigts ou un passe-main 13, sur la face extérieure 5.

Chaque dossard 10 comprend un bord médial 16 et un bord latéral 17. Les dossards 10 des deux branches 6, 7 sont des pièces distinctes, un espace 25 étant ménagé entre eux. Cet espace 25 est ménagé entre les bords médiaux 16 des dossards 10 et s'étend selon l'axe médian 2 de la ceinture 1. Chaque dossard 10 comprend en outre un bord supérieur 18 et un bord inférieur 19. En position d'utilisation, les dossards 10 sont placés de part et d'autre de la colonne vertébrale du porteur, les bords supérieurs 18 étant sensiblement dans un même plan horizontal, et les bords inférieurs 19 étant sensiblement dans un même plan horizontal.

Selon un exemple de réalisation, la partie principale 12 de chaque branche 6, 7 comporte deux bandes 20a, 20b élastiques qui sont superposées, sur une partie de leur surface, selon la direction antéro-postérieure X. avantageusement, ces deux bandes 20a, 20b sont identiques. Chaque branche 6, 7 peut être constituée de ces deux bandes 20a, 20b.

Par exemple, plus précisément, une bande supérieure 20a d'une branche 6, 7 est fixée au bord latéral 17 du dossard 10 de ladite branche, en partie supérieure de ce bord latéral 17, et s'étend jusqu'à la partie extrême latérale 11 de ladite branche, qui est de préférence agencée symétriquement par rapport à l'axe longitudinal 3. La bande supérieure 20a est ainsi inclinée vers le bas en s'éloignant du dossard 10.

En outre, une bande inférieure 20b de ladite branche est fixée au bord latéral 17 du dossard 10 de ladite branche, en partie inférieure de ce bord latéral 17, et s'étend jusqu'à la partie extrême latérale 11 de ladite branche, la bande inférieure 20b étant ainsi inclinée vers le haut en s'éloignant du dossard 10.

Chacune des bandes 20a, 20b peut avoir une largeur de l'ordre de 50 % à 70 % de la hauteur du dossard 10, selon l'axe médian 2.

Avec cet agencement, la partie principale 12 d'une branche 6, 7 présente la forme générale d'un trapèze avec un bord supérieur et un bord inférieur qui convergent l'un vers l'autre en direction de la partie extrême latérale 11. Les bords supérieur et inférieur peuvent être rectilignes. Un tel agencement simplifie considérablement la réalisation de la ceinture 1 en évitant la nécessité d'opérations de découpe, de couture et de bordage pour obtenir cette forme globale des branches. De plus, chaque branche 6, 7 comporte une zone de superposition 21 des bandes 20a, 20b qui s'étend sur l'ensemble de la partie principale 12, depuis le dossard 10 jusqu'à la partie extrême latérale 11. Avec la configuration décrite précédemment, cette zone de superposition 21 présente sensiblement la forme d'un triangle qui diverge en direction de la partie extrême latérale 11.

Chacune des bandes 20a, 20b peut présenter une nervosité supérieure à 175 cN/cm à 30% d'allongement, par exemple de l'ordre de 200 cN/cm, de sorte que chaque branche 6, 7 présente une nervosité de l'ordre de 400 cN/cm à 30% d'allongement.

La ceinture 1 comprend en outre un câble de serrage 30, qui est non-extensible. Le câble de serrage 30 présente une portion centrale de laçage 31 qui relie les deux dossards 10, ainsi que deux portions latérales 32. Chacune des portions latérales 32 est liée à l'une des extrémités latérales de la portion centrale de laçage 31.

Une traction sensiblement longitudinale exercée sur chaque portion latérale 32, dans des sens opposés, c'est-à-dire à l'opposé de l'axe médian 2, conduit au rapprochement mutuel des dossards 10, en d'autres termes à la diminution de la dimension de l'espace 25 selon l'axe longitudinal 3.

Le câble de serrage 30 présente un agencement symétrique par rapport à l'axe médian 2. Dans la réalisation représentée à titre d'exemple, le câble de serrage 30 présente en outre un agencement symétrique par rapport à l'axe longitudinal 3.

La portion centrale de laçage 31 comporte un brin longitudinal 33 qui passe dans un œillet 34 ménagé dans chacun des dossards 10, chacun desdits œillets 34 étant de préférence situé à proximité du bord supérieur 18 du dossard 10 correspondant et à proximité immédiate du bord médial 16 dudit dossard 10. Ce brin longitudinal 33 est prolongé de part et d'autre par un brin oblique 35. Chaque brin oblique 35 s'étend depuis un œillet 34 ménagé dans le dossard 10 d'une première branche 6, 7 jusqu'à un œillet de jonction 36 ménagé sur le dossard 10 de la deuxième branche 7, 6. L'œillet de jonction 36 est ménagé dans une patte 37 faisant saillie du dossard 10 de cette deuxième branche 7, 6, du côté de la partie principale 12 de cette deuxième branche 7, 6, à proximité du bord latéral 17 du dossard 10, sensiblement sur l'axe longitudinal 3.

Ainsi, chacun des brins longitudinaux 33 et des brins obliques 35 traverse l'espace 25 ménagé entre les dossards 10.

Cependant, de préférence, en dehors de l'espace 25, les brins obliques 35, qui sont situés du côté extérieur, sont masqués, par exemple entre le dossard 10 correspondant et un pan de tissu cousu sur le dossard 10 pour former un logement de réception du brin oblique 35. Sur les figures 4, 6, 7, 8, 9 montrant la face extérieure 5 de la ceinture 1, sont représentés les bords 8 dudit pan de tissu, qui peut être en forme de V. De plus, chacune des pattes 37 est de préférence logée entre les bandes 20a, 20b, dans la zone de superposition 21.

Dans la réalisation représentée, la portion centrale de laçage 31 comporte une partie qui est le symétrique de la partie décrite ci-dessus par rapport à l'axe longitudinal 3. Ainsi, la portion centrale de laçage 31 du câble de serrage 30 comporte deux parties, de préférence symétriques, agencées de part et d'autre de l'axe longitudinal 3, chaque partie comportant un brin longitudinal 33 prolongé de part et d'autre par un brin oblique 35, l'ensemble des brins obliques formant un losange.

Le brin oblique 35 supérieur et le brin oblique 35 inférieur d'une même branche 6, 7 se rejoignent dans l'œillet de jonction 36 correspondant. A partir de l'œillet de jonction 36, lesdits brins obliques 35 sont prolongés, dans le sens de l'éloignement par rapport à l'axe médian 2, par une portion qui passe dans un anneau 39 et qui est repliée sur elle-même. Ladite portion est donc formée de deux brins axiaux 38 qui comprennent chacun une extrémité médiale liée à un brin oblique 35 et une portion latérale située au niveau de l'anneau 39, les extrémités latérales des deux brins axiaux 38 étant solidaires l'une de l'autre. Ces brins axiaux 38 suivent ainsi une trajectoire sensiblement longitudinale entre les bandes 20a, 20b, dans la zone de superposition 21, en s'éloignant de l'axe médian 2, jusqu'à l'anneau 39 également situé dans la zone de superposition 21.

Selon un mode de réalisation, les deux brins longitudinaux 33, les quatre brins obliques 35 et les quatre brins axiaux 38 peuvent former une première boucle fermée.

Enfin, au niveau de chaque branche 6, 7, le câble de serrage 30 comprend une portion terminale 40 qui passe dans l'anneau 39 et qui est repliée sur elle-même. Plus spécifiquement, la portion terminale 40 est formée d'un premier brin terminal 40a et d'un deuxième brin terminal 40b. Le premier brin terminal 40a comprend une extrémité médiale située au niveau de l'anneau 39 et une extrémité latérale qui est fixée sur la partie extrême latérale 11 correspondante. Le deuxième brin terminal 40b comprend une extrémité médiale située au niveau de l'anneau 39 et une extrémité latérale qui est fixée à un organe de préhension 41, accessible au porteur depuis le côté extérieur de la ceinture 1. Les extrémités latérales des deux brins terminaux 40a, 40b sont solidaires l'une de l'autre.

Chacun des brins terminaux 40a, 40b s'étend sensiblement longitudinalement entre les bandes 20a, 20b, dans la zone de superposition 21, en s'éloignant de l'axe médian 2, jusqu'à un œillet terminal 42. Les brins terminaux 40a, 40b sont engagés dans l'oeillet terminal 42, et traversent ainsi la bande 20a située du côté extérieur de la ceinture 1. La portion terminale 40 forme ainsi une deuxième boucle fermée qui est distincte de la première boucle et qui forme la portion latérale 32 du câble de serrage 30.

La ceinture 1 comprend en outre un dispositif de liaison 50 des dossards 10 entre eux. Le dispositif de liaison 50 est agencé entre les dossards 10 et fixé à chacun des dossards 10. Il est distinct du câble de serrage 30.

Le dispositif de liaison 50 est élastiquement déformable selon l'axe longitudinal 3. Par ailleurs, il possède dans la direction orthogonale à l'axe longitudinal 3, c'est-à-dire parallèlement à l'axe médian 2 de la ceinture 1, une nervosité suffisamment importante pour empêcher sensiblement le décalage d'un dossard 10 par rapport à l'autre parallèlement à l'axe médian 2.

A titre d'exemple, le dispositif de liaison 50 peut présenter une nervosité comprise entre 50 et 150 cN/cm à 30% d'allongement, par exemple de l'ordre de 100 cN/cm à 30% d'allongement.

Le dispositif de liaison 50 est de préférence agencé sensiblement dans la partie centrale de la ceinture 1, selon l'axe médian 2. Ainsi, la portion centrale de laçage 31 du câble de serrage 31 peut comporter deux parties agencées de part et d'autre du dispositif de liaison 50, selon l'axe médian 2. Le dispositif de liaison 50 peut s'étendre sur une hauteur comprise entre 25 % et 45 %, par exemple de l'ordre du tiers, de la hauteur des dossards 10, le long de l'axe médian 2. Le dispositif de liaison 50 est ici agencé du côté extérieur de la ceinture 1, mais il pourrait en variante être agencé du côté intérieur.

Selon un mode de réalisation, tel qu'illustré sur les figures 4 à 7, le dispositif de liaison 50 comprend deux sangles 51 allongées indépendantes l'une de l'autre, c'est-à-dire non attachées l'une à l'autre. Chaque sangle 51 est disposée en oblique et possède une première extrémité 52 fixée à l'un des dossards 10 et une deuxième extrémité 53 fixée à l'autre des dossards 10. Les deux sangles 51 forment ainsi une croix.

On décrit à présent le comportement de la ceinture 1 lors de sa mise en place sur un porteur.

A l'état non contraint, comme illustré sur les figures 4 et 5, le dispositif de liaison 50 est au repos, non étendu. Du fait de son élasticité et de sa nervosité, il maintient les dossards 10 l'un par rapport à l'autre ceux-ci étant espacés selon l'axe longitudinal 3 d'une première distance d1. De plus, les dossards 10 sont situés à la même hauteur l'un par rapport à l'autre, c'est-à-dire que leurs bords supérieurs 18, d'une part, et leurs bords inférieurs 19, d'autre part, sont situés sensiblement dans deux plans parallèles. Selon le dimensionnement du dispositif de liaison 50, la distance d1 peut être nulle.

Par ailleurs, aucune traction extérieure n'est exercée sur les branches 6, 7 ni sur le câble de serrage 30. La ceinture 1 présente alors une longueur L1, le long de l'axe longitudinal 3.

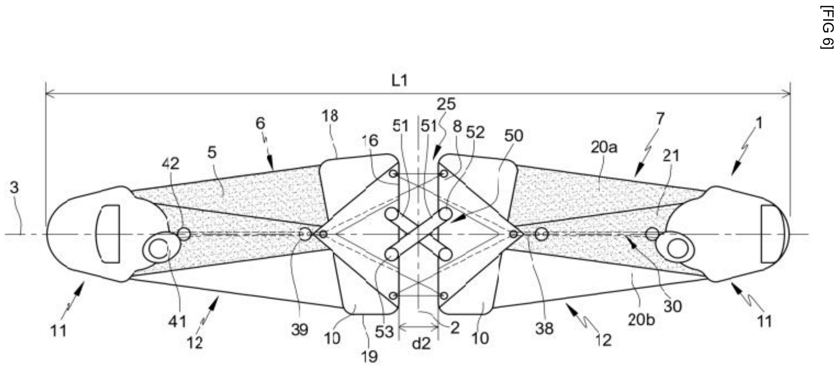

A partir de cet état non contraint, et préalablement à la mise en place de la ceinture 1, les dossards 10 sont espacés l'un de l'autre selon l'axe longitudinal 3, jusqu'à être espacés d'une deuxième distance d2 > d1. De préférence, la distance d2 est au maximum de 6 cm environ. La ceinture 1 peut être conçue pour procurer une valeur maximale possible d'écartement, par exemple du fait de la longueur de câble disponible une fois que les organes de préhension 41 sont en butée contre l'oeillet terminal 42, comme illustré sur la figure 6. Les organes de préhension 41 peuvent être accrochés, de façon détachable, sur les parties extrêmes latérales 11 de la ceinture 1, typiquement via un système de type Velcro ^{®}.

L'espacement mutuel des dossards 10 est rendu possible par le fait que le dispositif de liaison 50 est élastiquement déformable. Le dispositif de liaison 50 se trouve alors dans un état étendu, illustré sur la figure 6.

Lors du passage du dispositif de liaison 50 de l'état non contraint à l'état étendu, la ceinture 1 conserve de préférence la même longueur L1.

Un utilisateur peut alors placer la ceinture 1 autour de son tronc, le dispositif de liaison 50 étant dans cet état étendu. L'utilisateur peut exercer une légère traction sur les branches 6, 7, sensiblement longitudinalement et dans des sens opposés, les branches 6, 7 pouvant ainsi être allongées par rapport à l'état non contraint. Il est cependant précisé que l'essentiel de la force de serrage n'est pas obtenu lors de cette première étape de mise en place de la ceinture 1, mais dans un deuxième temps, par rapprochement mutuel des dossards 10 comme on le décrit ci-après. Ainsi, une fois en place autour de l'utilisateur, la ceinture 1 présente une longueur L2 qui est par exemple comprise entre L1 et 1,1 x L1. En outre, à ce stade, la partie principale 12 de chacune des branches 6, 7 présente une longueur L (avec une ceinture 1 mise en place de façon symétrique).

Une fois que le porteur a assemblé les parties extrêmes latérales 11 l'une à l'autre grâce aux moyens d'accrochage 14, 15, avec éventuellement un premier serrage d'amplitude assez faible, le porteur peut procéder à un serrage additionnel au moyen du câble de serrage 30. Ceci correspond à une deuxième étape de mise en place de la ceinture 1.

Pour ce faire, le porteur détache les organes de préhension 41 et exerce une traction sensiblement longitudinale sur les portions latérales 32 du câble de serrage 30, dans des sens opposés. Lors de cette traction, les parties extrêmes latérales 11 des branches 6, 7 de la ceinture 1 restent immobiles l'une par rapport à l'autre. Le fait que le câble de serrage 30 comporte des brins en zigzag passant dans des œillets et un anneau permet une démultiplication de la force de serrage.

De façon concrète, une traction est exercée sur les organes de préhension 41, c'est-à-dire sur les brins terminaux 40a, 40b et les anneaux 39 puis, via les brins axiaux 38, sur les brins obliques 35. Lesdits brins obliques 35 coulissent dans les œillets 34 et, par conséquent, la longueur des brins longitudinaux 33 diminue. En d'autres termes, lors de cette traction, les dossards 10 se rapprochent l'un de l'autre selon l'axe longitudinal 3, jusqu'à ce que le dispositif de liaison 50 atteigne l'état serré souhaité. Lorsque le serrage souhaité est obtenu, les organes de préhension 41 sont fixés chacun à la partie extrême latérale 11 correspondante, par exemple par un accrochage de type Velcro^{®}.

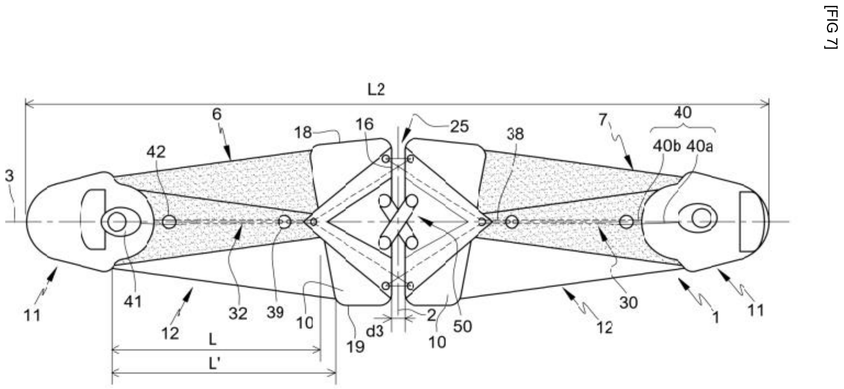

A l'état serré, comme illustré sur la figure 7, la ceinture 1 présente le long de l'axe longitudinal 3 une longueur L2 inchangée par rapport à l'état étendu. Cependant, les dossards 10 sont espacés selon l'axe longitudinal 3 d'une troisième distance d3 telle que d1 ≤ d3 < d2. Ainsi, chacune des parties principales 12 des branches 6, 7 est davantage étirée par rapport à l'état étendu de la ceinture 1, ce qui produit le serrage additionnel recherché. En pratique, et comme illustré sur la figure 7, la longueur de la partie principale 12 de chacune des branches 6, 7 a augmenté de L à L' > L.

Lors du passage de l'état étendu à l'état serré, le dispositif de liaison 50 tend à revenir vers son état de repos par retour élastique selon l'axe longitudinal 3. En revanche, selon l'axe médian 2, du fait de sa nervosité, le dispositif de liaison 50 maintient les dossards 10 sensiblement à la même hauteur, en empêchant le décalage de l'un des dossards 10 par rapport à l'autre parallèlement à l'axe médian 2. Le dispositif de liaison 50 fournit donc un accompagnement du mouvement de serrage et un guidage de ce mouvement, permettant de contrôler ce mouvement, en particulier en restreignant ou en empêchant le déplacement mutuel des dossards 10 parallèlement à l'axe médian 2. Il est à noter que le risque de décalage vertical des dossards 10 l'un par rapport à l'autre, au cours du serrage, est d'autant plus important que les dossards 10 sont initialement écartés d'une distance importante.

Comme illustré sur la figure 7, à l'état serré, le dispositif de liaison 50 peut être déformé longitudinalement par rapport à sa géométrie à l'état non contraint. En variante, et selon le serrage appliqué, le dispositif de liaison 50 pourrait être moins déformé, et notamment retrouver la géométrie qu'il avait à l'état non contraint.

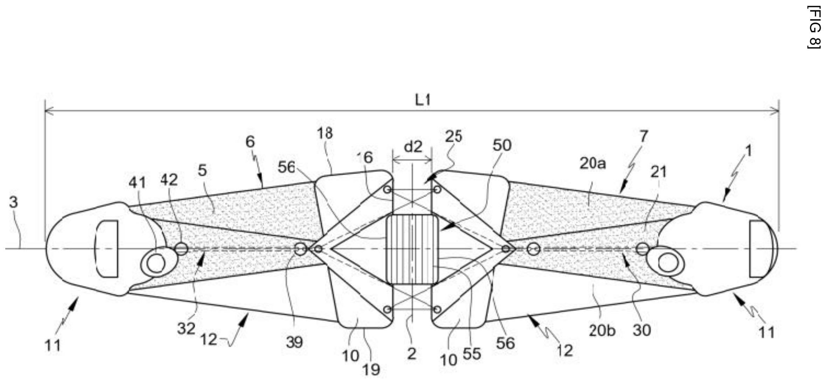

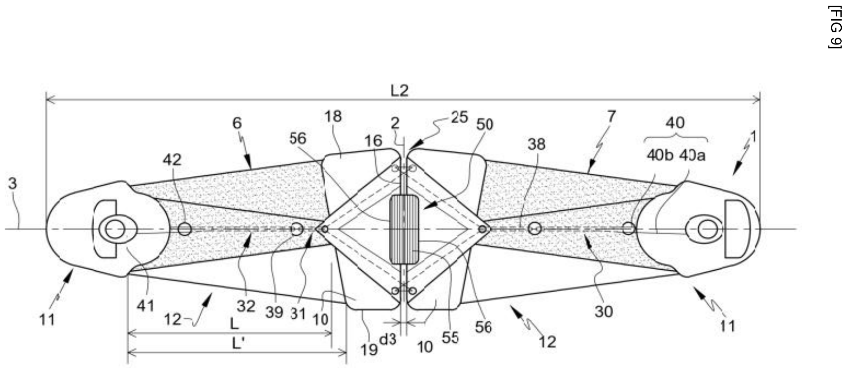

Selon un autre mode de réalisation, tel qu'illustré sur les figures 8 et 9, le dispositif de liaison 50 comprend un élément 55 globalement rectangulaire possédant, à l'état non contraint, deux grands côtés 56 qui s'étendent sensiblement orthogonalement à l'axe longitudinal 3 et qui sont fixés chacun à un dossard 10.

De façon similaire au mode de réalisation précédemment décrit, à partir d'un état non contraint (non représenté) où le dispositif de liaison 50 est au repos, les dossards 10 étant espacés selon l'axe longitudinal 3 d'une première distance d1 et la ceinture 1 présentant une longueur L1, le dispositif de liaison 50 peut passer à un état étendu, illustré sur la figure 8.

Le passage de l'état non contraint à l'état étendu est la conséquence d'un écartement mutuel des dossards 10.

Ainsi, à l'état étendu, comme illustré sur la figure 8, la ceinture 1 présente le long de l'axe longitudinal 3 une longueur L1 inchangée par rapport à l'état non contraint. En outre, les dossards 10 sont espacés selon l'axe longitudinal 3 d'une deuxième distance d2 > d1.

Lors du passage de l'état non contraint à l'état étendu, le dispositif de liaison 50 se déforme élastiquement selon l'axe longitudinal 3, autorisant l'espacement mutuel des dossards 10.

Ensuite, le porteur place et referme la ceinture 1 autour de son tronc, éventuellement avec une légère traction conduisant à un allongement de la ceinture 1 jusqu'à une longueur L2, le dispositif de liaison 50 restant dans l'état étendu.

Puis le porteur peut exercer une traction sensiblement longitudinale sur les portions latérales 32 du câble de serrage 30, dans des sens opposés, afin d'obtenir le serrage souhaité. Lors de cette traction, les parties extrêmes latérales 11 des branches 6, 7 de la ceinture 1 restent immobiles l'une par rapport à l'autre.

Comme précédemment décrit, cette traction conduit au rapprochement mutuel des dossards 10.

A l'état serré, comme illustré sur la figure 9, la ceinture 1 présente le long de l'axe longitudinal 3 une longueur L2 inchangée par rapport à l'état étendu. Les dossards 10 sont espacés selon l'axe longitudinal 3 d'une troisième distance d3 telle que d1 ≤ d3 < d2, et chacune des parties principales 12 des branches 6, 7 est davantage étirée par rapport à l'état étendu de la ceinture 1.

Lors du passage de l'état étendu à l'état serré, le dispositif de liaison 50 tend à revenir vers son état de repos par retour élastique selon l'axe longitudinal 3. En revanche, selon l'axe médian 2, du fait de sa nervosité, le dispositif de liaison 50 maintient les dossards 10 sensiblement à la même hauteur, en empêchant le décalage de l'un des dossards 10 par rapport à l'autre parallèlement à l'axe médian 2. Le dispositif de liaison 50 fournit donc un d'accompagnement du mouvement de serrage et un guidage de ce mouvement, en particulier en restreignant ou en empêchant le déplacement mutuel des dossards 10 parallèlement à l'axe médian 2.

Il va de soi que l'invention n'est pas limitée aux modes de réalisation décrits ci-dessus à titre d'exemples mais qu'elle comprend tous les équivalents techniques et les variantes des moyens décrits ainsi que leurs combinaisons.

## Revendications

1. Ceinture (1) de soutien lombaire présentant un axe médian (2) de symétrie et un axe longitudinal (3) orthogonal à l'axe médian (2), la ceinture (1) comportant deux branches (6, 7) destinées à être positionnées de part et d'autre de la partie inférieure du tronc d'un porteur, chaque branche (6, 7) comprenant une partie extrême médiale (9) comportant un dossard (10) destiné à être placé contre le dos du porteur, une partie extrême latérale (11), et une partie principale (12) qui est élastiquement extensible longitudinalement et qui relie le dossard (10) et la partie extrême latérale (11), les dossards (10) des deux branches (6, 7) étant des pièces distinctes, les parties extrêmes latérales (11) étant pourvues de moyens d'accrochage complémentaires (14, 15) pour pouvoir être assemblées lorsqu'elles sont placées l'une sur l'autre en regard de la zone abdominale du porteur, la ceinture (1) comprenant en outre un câble de serrage (30), ledit câble de serrage (30) étant non-extensible et présentant d'une part une portion centrale de laçage (31) qui relie les deux dossards (10), et d'autre part deux portions latérales (32) reliées chacune à l'une des extrémités latérales de la portion centrale de laçage (31), une traction sensiblement longitudinale exercée sur chaque portion latérale (32), dans des sens opposés, conduisant au rapprochement mutuel des dossards (10), **caractérisée en ce que** la ceinture (1) comprend en outre un dispositif de liaison (50) des dossards (10) entre eux, distinct du câble de serrage (30), ledit dispositif de liaison (50) étant agencé entre les dossards (10) et fixé à chacun des dossards (10), ledit dispositif de liaison (50) étant élastiquement déformable selon l'axe longitudinal (3) et possédant dans la direction orthogonale à l'axe longitudinal (3) une nervosité suffisamment importante pour empêcher sensiblement le décalage d'un dossard (10) par rapport à l'autre parallèlement à l'axe médian (2) de la ceinture (1).

2. Ceinture selon la revendication 1, **caractérisée en ce que** la nervosité du dispositif de liaison (50) est inférieure à la nervosité de chacune des branches (6, 7).

3. Ceinture selon la revendication 1 ou 2, **caractérisée en ce que** le dispositif de liaison (50) peut passer :
- d'un état non contraint, dans lequel les dossards (10) sont espacés selon l'axe longitudinal (3) d'une première distance d1 ;
- à un état étendu, à la suite d'un écartement mutuel des dossards (10) selon l'axe longitudinal (3) , état étendu dans lequel les dossards (10) sont espacés selon l'axe longitudinal (3) d'une deuxième distance d2 supérieure à la première distance d1, le dispositif de liaison (50) étant élastiquement déformé,
- puis à un état serré, à la suite d'une traction sensiblement longitudinale exercée sur les portions latérales du câble de serrage (30), dans des sens opposés, état serré dans lequel les dossards (10) sont espacés selon l'axe longitudinal (3) d'une troisième distance d3 telle que d1 ≤ d3 < d2.

4. Ceinture selon l'une des revendications 1 à 3, **caractérisée en ce que** le dispositif de liaison (50) est agencé du côté extérieur de la ceinture (1), c'est-à-dire le côté tourné à l'opposé du porteur, en position d'utilisation.

5. Ceinture selon l'une des revendications 1 à 4, **caractérisée en ce que** le dispositif de liaison (50) est agencé sensiblement dans la partie centrale de la ceinture (1), selon l'axe médian (2).

6. Ceinture selon la revendication 5, **caractérisée en ce que** la portion centrale de laçage (31) du câble de serrage (30) comporte deux parties agencées de part et d'autre du dispositif de liaison (50), selon l'axe médian (2).

7. Ceinture selon l'une des revendications 1 à 6, **caractérisée en ce que** le dispositif de liaison (50) s'étend sur une hauteur comprise entre 25 % et 45 %, par exemple de l'ordre du tiers, de la hauteur des dossards (10), le long de l'axe médian (2).

8. Ceinture selon l'une des revendications 1 à 7, **caractérisée en ce que** le dispositif de liaison (50) comprend deux sangles (51) allongées indépendantes l'une de l'autre, chaque sangle (51) étant disposée en oblique et possédant une première extrémité (52) fixée à l'un des dossards (10) et une deuxième extrémité (52) fixée à l'autre des dossards (10), les deux sangles (51) formant une croix.

9. Ceinture selon l'une des revendications 1 à 7, **caractérisée en ce que** le dispositif de liaison (50) comprend un élément (55) globalement rectangulaire possédant, à l'état non contraint, deux grands côtés (56) qui s'étendent sensiblement orthogonalement à l'axe longitudinal (3) et qui sont fixés chacun à un dossard (10).

10. Ceinture selon l'une des revendications 1 à 9, **caractérisée en ce que** la partie principale (12) de chaque branche (6, 7) comporte deux bandes (20a, 20b) élastiques qui sont superposées, sur une partie de leur surface, selon la direction antéro-postérieure (X) et **en ce que**, pour chaque branche (6, 7), la portion latérale (32) du câble de serrage (30) est agencée entre les deux bandes (20a, 20b) élastiques selon la direction antéro-postérieure (X), à l'exception d'une partie extrême latérale (40) du câble de serrage (30) qui traverse une bande (20a, 20b) pour être située du côté extérieur de la ceinture (1).
